Europäisches Patentamt

European Patent Office

Office européen des brevets

(19)

(11) Publication number: **0 140 689**
A2

(12) **EUROPEAN PATENT APPLICATION**

(21) Application number: 84307400.6

(22) Date of filing: 26.10.84

(51) Int. Cl.⁴: **C 12 N 5/00**, C 12 N 15/00,
C 12 P 21/00, G 01 N 33/577
// (C12P21/00, C12R1:91)

(30) Priority: 27.10.83 US 545897

(43) Date of publication of application: 08.05.85
Bulletin 85/19

(84) Designated Contracting States: **BE DE FR GB IT LU NL
SE**

(71) Applicant: **BOARD OF REGENTS THE UNIVERSITY OF
TEXAS SYSTEM, 201 West 7th Street, Austin
Texas 78701 (US)**

(72) Inventor: **Baseman, Joel B., 11010 Whisper Hollow, San
Antonio Texas 78230 (US)**

(74) Representative: **Clifford, Frederick Alan et al, MARKS &
CLERK 57/60 Lincoln's Inn Fields, London WC2A 3LS
(GB)**

(54) Hybrid cell lines producing monoclonal antibodies directed against treponema and use thereof for immuno-diagnosis of treponemal infections.

(57) The present invention provides hybrid cell lines producing monoclonal antibodies reactive with the human pathogenic treponemes; T. pallidum and T. pertenue, but not reactive with the treponemes which are not pathogenic to man. T. hyodysenteriae and T. phagedenis biotype Reiter. Further, there is provided hybrid cell lines which have specificity for the immunodominant P6 protein shared by the pathogens T. pallidum and T. pertenue. Diagnostic analysis of the pathogenic treponeme, T. pallidum, from infected tissue and serum is accomplished using a selected monoclonal antibody composition, termed 13F₃.

## HYBRID CELL LINES PRODUCING MONOCLONAL ANTIBODIES DIRECTED AGAINST TREPONEMA AND USE THEREOF FOR IMMUNO-DIAGNOSIS OF TREPONEMAL INFECTIONS

The present invention relates to hybridomas producing monoclonal antibodies specific for Treponema determinants. More specifically, this invention relates to hybridomas and monoclonal antibodies produced therefrom having cross reactive specificity for determinants common to pathogenic Treponema species and their use in diagnostic assays.

Treponematoses are a significant worldwide human health problem. There are four distinct and clinically dissimilar diseases, which are caused by three species of treponemes. Treponema pallidum is the etiological agent of venereal syphilis as well as the non-venereal endemic syphilis (bejel). T. pertenue is the agent responsible for yaws, and T. carateum causes pinta. Although the clinical syndromes are distinct, biological differences between the causative agents have never been adequately described. In contrast to syphilis, which is sexually transmitted, yaws and pinta appear to be transmitted by close personal contact. This transmission occurs under conditions in which abrasions of skin or mucous membranes permit entry of treponemes shed from exuding lesions. Until recently, none of the pathogenic treponemes has been cultured in vitro; however, T. pallidum and T. pertenue are routinely passaged in rabbits. T. carateum however cannot be passaged in rabbits. Upon isolation of these spirochetes from infected rabbit tissue, they so closely resemble each other that they cannot be distinguished either morphologically or serologically. The fluorescent antibody and treponemal immobilization tests exhibit no differences when yaws sera or syphilitic sera are reacted against either T. pallidum or T. pertenue. However, the type of lesion, and the period required for development of orchitis differ for these two microorganisms. T. pallidum requires 8-12 days to establish orchitis in the intratesticular infected rabbit, while T. pertenue requires 26-30 days.

Since serological testing has failed to distinguish between these treponemes, biochemical studies have been undertaken. When DNA from T. pallidum and T. pertenue were examined by Cot analysis of DNA-DNA hybridization, they were found to be genetically indistinguishable.

There is therefore an inconsistency between the clinical data, presenting two distinct diseases, and the biochemical data which indicate that these microorganisms are virtually indistinguishable. The present invention provides a mechanism to compare the antigenic peptides and the intrinsic and surface-labeled protein profiles common to the pathogenic Treponema species.

It therefore is highly desirable to provide hybrid cell lines and monoclonal antibodies to pathogenic Treponema antigens. Such antibodies would be important in the differential diagnosis of treponemal disease in humans, in the purification of specific immunogens for subsequent use as vaccines, and studying the structure and function of immunogenic components of virulent treponemes.

0140689

In accordance with the present invention, continuous hybridoma cell lines are established which elaborate and secrete highly specific and homogenous monoclonal antibodies crossreactive with antigenic determinants common to the pathogenic treponemes, namely Treponema pallidum and Treponema pertenue.

Further, the monoclonal antibodies according to this invention provide a diagnostic reagent useful for the immunochemical diagnosis of treponemal infections pathogenic to man.

The following discussion is in terms of the preferred embodiments of this invention, which represents the best mode known to the Applicant at the time of this application.

Antibodies are normally synthesized by lymphoid cells derived from B lymphocytes of bone marrow.  The great diversity of antibody specificities is accomplished by immunoglobulin molecules having many structural features in common.  Individual antibody molecules of heterogeneous binding specificity differ in their detailed amino acid sequences and even antibodies of the same specificity are usually a mixture of immunoglobulins having different amino acid sequences, although such sequences may be substantially homologous.  The terms "antibody" and "immunoglobulin" are used interchangeably herein.

Individual lymphocytes produce immunoglobulin of a single amino acid sequence.  Lymphocytes cannot be directly cultured to produce their specific antibody. However, Kohler, et al, Nature 256:495 (1975) demonstrated that a process of somatic cell fusion, specifically between a lymphocyte and a myeloma cell, could yield hybrid cells which grow in culture and produce a specific antibody. Myeloma cells are lymphocyte tumor cells which, depending upon the cell strain, frequently produce an antibody themselves, moreover some "non-producing" strains are known.

The hybrid resulting from somatic fusion of a lymphocyte and a myeloma cell is termed a "hybridoma" cell herein and in the art generally. In a typical fusion procedure, spleen lymphocytes from an animal immunized against a chosen antigen are fused with myeloma cells. The resulting hybridomas are then dispersed in a series of separate culture tubes or microtiter plate wells to screen for cultures producing a desired antibody. Positive cultures are further diluted to obtain colonies arising from a single cell (clones). The clones are again screened for production of the desired antibody. Antibody produced by a cloned hybridoma is termed "monoclonal" herein and in the art.

Monoclonal antibodies are highly specific, being directed against a single antigen only. Furthermore, in contrast to conventional antibody preparations which typically include different antibodies directed against different sets of determinants on the same antigen, monoclonal antibodies are directed only against a single determinant on the antigen. Monoclonal antibodies are useful to improve the selectivity and specificity of diagnostic and analytical assay methods using antigen-antibody binding. A second advantage of monoclonal antibodies is provided by the fact that they are synthesized in pure form by the hybridoma culture uncontaminated by other immunoglobulins. Monoclonal antibodies may be prepared from supernatants of cultured hybridoma cells or from ascites fluid induced by intraperitoneal inoculation of hybridoma cells into mice.

In accordance with the processes to develop the hybrid cell lines and monoclonal antibodies of this invention, test animals are stimulated for antibody production by immunization regimens using antigen preparations of _Treponema pallidum_. For example, immunization of test animals can be accomplished following subcutaneous inoculation of live organisms in the hind quarters of BALB/c mice or via intraperitoneal injection of disrupted membrane material or purified protein preparations representative of _T. pallidum_ immunodominant antigens. In all cases, Applicant has directed the preferred embodiment to immunization of mice with a heterogeneous composition of parasite materials, thereby providing a complex array of antigenic determinants.

The route and schedule of immunization of the host animal is in keeping with established and conventional techniques for antibody stimulation and production. Applicant has employed mice as a test model although it is contemplated that any mammalian subject, including human subjects or antibody-producing cells therefrom, can be manipulated to serve as the basis for production of hybrid cell lines.

After immunization, immune lymphoid cells are fused with myeloma, plasmacytoma, or hybridoma cells (hereinafter referred to collectively as myeloma cells) to generate a hybrid cell line which can be cultivated and subcultivated indefinitely, to produce large quantities of monoclonal antibodies.

For purposes of this invention, the immune lymphoid cells selected for fusion are lymphocytes and their normal differentiated progeny, taken either from lymph node tissue or spleen tissue from immunized animals. Applicant prefers to employ immune spleen cells, since they offer a more concentrated and convenient source of antibody producing cells with respect to the mouse system.

The myeloma cells provide the basis for continuous propagation of the fused hybrid. Myeloma cells are tumor cells derived from plasma cells which show preference for bone marrow. Plasmacytoma cells are neoplastic cells derived from plasma cells. In particular, Applicant prefers to use lymphocyte hybridoma cells which secrete no immunoglobulin. Lymphocyte hybridoma cells are cells generated by the fusion of myeloma or plasmacytoma cells with normal differentiated lymphoid cells. Myeloma, plasmacytoma, and hybridomas can be selected to be devoid of immunoglobulin synthesis.

The particular species of animal from which the myeloma and immunized antibody producing cells are derived are not critical, in that it is possible to fuse cells of one species with another. However, it is preferred that the source of immunized antibody producing cells and myeloma be from the same species.

Generally the fusion techniques employed are according to the procedures set out by Kohler et al, <u>Eur. J. Immunol.</u> 6:11-19 (1976) and Kennett et al, <u>Lymphocyte Hybridomas - Current Topics in Microbiology and Immunology</u> B1:77-91 (1978) Springer-Verlag, New York. Fusion is generally accomplished by adding a suspension of antibody producing cells to the myeloma cells in growth medium which is centrifuged to form a pellet prior to addition of the fusing agent, polyethylene glycol.

The fused hybrids are next screened for antibody production specific for antigens common to the pathogenic treponemes. The monoclonal antibodies obtained according to preferred examples include antibodies with individual specificity for the antigenic protein membrane components common to each of the virulent Treponema, namely <u>T. pallidum</u> and <u>T. pertenue</u>. The monoclonal antibodies of this invention, however, have neglible specificity for antigenic determinants not shared by the avirulent Treponema species.

The hybridomas which secrete antibody specific for antigens shared by the pathogenic Treponema are cultured to establish a continuous cell line with stable genetic coding. These cell lines can be stored and preserved in any of a number of conventional ways, including freezing and storage under liquid nitrogen. Frozen cell lines can be revived and cultured indefinitely with resumed synthesis and secretion of monoclonal antibodies specific for shared antigens of pathogenic Treponema. The secreted antibody is recovered from tissue culture supernatant by conventional precipitation, ion exchange, affinity chromatography, or the like. The recovered antibody can be freeze dried and stored under refrigeration for at least several weeks without significant loss of activity.

The following examples are offered to illustrate a particular embodiment of the invention but they are not intended to limit it.

A. <u>Preparation of Antigens</u>
<u>T. pallidum</u> (Nichols strain) and <u>T. pertenue</u> (Gautier strain) were obtained from the Center for Disease Control, Atlanta, Georgia, and were maintained by routine passage in rabbits. Stock solutions of treponemes were cryopreserved at -80° as described in <u>Cryobiology</u> 9:404-10 (1972). Treponemes were harvested from infected rabbit testes as previously outlined in <u>Infection and Immunity</u> 26:1048-56 (1979). Treponemes were removed from minced testicular tissue excised at peak orchitis (11-14 days,

post-inoculation with <u>T. pallidum</u> and 26-30 days, post-inoculation with <u>T. pertenue</u>) by shaking in 15 ml of a salts-glucose treponemal medium under reducing conditions for 15 minutes at room temperature. A treponemal suspension of $1.5 \times 10^8$ to $4.0 \times 10^8$ organisms per ml was clarified twice at 500 x g for 15 minutes followed by removal of cellular debris by centrifugation on a cushion of 0.8% Methacel (Dow Chemical Co., Midland, MI)-50% Hypaque (Winthrop Laboratories, New York, NY) at 800 x g for 20 minutes at room temperature. Next the respective treponeme was pelleted at 17,000 x g and washed twice in PBS (137 mM NaCl, 2.7 mM KCl, 4.6 mM $Na_2PO_4$, and 1.5 mM $KH_2PO_4$) to remove loosely-bound host plasma proteins associated with the treponemal outer envelope. Organisms were then resuspended in PBS to the desired concentration.

B.  <u>Preparation of Lymphocytes for Fusion</u>
The treponemes ($6 \times 10^8$ <u>T. pallidum</u> in 0.5 ml saline) were emulsified 1:1 (vol:vol) in Freund's complete adjuvant. Organisms were administered intramuscularly (i.m., 0.1 ml), subcutaneously (s.c., 0.1 ml), and intraperitoneally (i.p., 0.3 ml) into BALB/c female mice (3-6 weeks old). Each mouse received a total of $3 \times 10^8$ <u>T. pallidum</u>. On days 7 and 21 mice were boosted with $3 \times 10^8$ treponemes emulsified in Freund's incomplete adjuvant as above. Four days after the final immunization, the spleen was removed and spleen cells isolated for use in hybridoma construction.

C.  <u>Construction of Hybridomas</u>
Hybridomas were produced by fusing spleen cells from the immunized mice with murine SP2/0 -Ag14 hybridoma cells (SP2/0 hereinafter) using a modification of the basic procedure of Oi and Herzenberg, <u>Immunoglobulin-Producing Hybrid Cell Lines</u>, In Selected Methods in Cellular Immunology, B.B. Mishell and S.M. Shiigi, eds., pp. 351-371, W.H. Freeman and Co., 1980, San Francisco.

Suitable cell lines were obtained from Ed Hayes, Duke University and S. Robertson (The University of Texas Health Science Center at Dallas) and are as originally set forth by Schulman et al, Nature 276:269-270 (1978). The SP2/0 hybridoma cell line is a hybrid cell line derived from SP2/HGLK formed as a hybrid between a BALB/c spleen cell and the myeloma cell lines X63-Ag8. This cell line synthesizes no immunoglobulin chains, lacks the enzyme hypoxanthine guanine phosphoribosyl-transferase (HGPRT), is resistant to 8-azaguanine, and dies in the presence of Littlefield's hypoxanthine-aminopterin-thymidine (HAT) selection medium. SP2/0 cells were grown in Dulbecco's Modified Eagle's Medium (DMEM) (Microbiological Associates, Walkersville, MD) supplemented with 15% (vol/vol) heat-inactivated fetal calf serum (Microbiological Associates), 2 mM L-glutamine, and 50 units/ml penicillin and 50µg/ml streptomycin. SP2/0 cells were grown in this medium containing 8-azaguanine (20 µg/ml immediately prior to use in hybridization experiments to ensure that no HGPRT-positive revertants were present in the cell culture.

Spleens were removed aseptically from immunized mice and teased apart gently with forceps to prepare a single cell suspension in serum-free Dulbecco's Modified Eagle's Medium (DMEM). SP2/0 cells were harvested in the logarithmic phase of growth and both cell types were collected by centrifugation at 270 x g for 10 minutes at 8°C and washed three times with DMEM. Total cell numbers were determined with a Neubauer Counting hemocytometer and viability was measured by trypan blue exclusion.

Approximately $10^8$ spleen cells were mixed together with SP2/0 cells in a 50 ml conical tube at a ratio of 7 viable spleen cells per viable SP2/0 cell and the resultant cell suspension was collected in a pellet by centrifugation at 400 x g for 10 minutes. The supernatant medium was removed and the tube containing the cell pellet was placed in a 37°C water bath for 1 minute.

Cell fusion was initiated by the dropwise addition of a 1 ml portion of prewarmed (37°C) 50% (wt/vol) solution of polyethylene glycol (PEG 1000; ATCC) in DMEM to the cell pellet with gentle stirring over a 1 minute period. The suspension was stirred an additional minute. Then, 2 ml of serum free DMEM were added over an additional 2 minute period. Finally, 7 ml (per 1-2 x $10^8$ spleen cells) of prewarmed DMEM supplemented with 20% fetal bovine serum (tested for hybridoma growth, Microbiological Associates) were added over 2-3 minutes. The cells were then centrifuged at 400 x g for 10 minutes and cells resuspended to 2-4 x $10^6$ per ml in DMEM supplemented with 3 x $10^{-6}$ M glycine.

Fifty microliters of this suspension were aliquoted into each well of 96-well microtiter tissue culture plates (Bellco, Vineland, NJ) containing 1 x $10^5$ normal BALB/c spleen feeder cells in 50 μl of DMEM plus glycine.

One day post fusion, 100 μl of DMEM plus glycine supplemented with 0.2 mM hypoxanthine, 0.7 mM aminopterin; and 32 mM thymidine (HAT selection medium) were added to each well. The cells where then incubated at 37°C in a humidified incubator containing a 7% $CO_2$ atmosphere for 7-10 days to permit growth of hybrid cell lines.

The unfused SP2/0 cells died in HAT within 24-48 hours. Cell growth in HAT medium is indicative of suc- cessful hybridization. Wells which contained growing clones were assayed to detect monoclonal antibodies directed against antigenic determinants common to the pathogenic Treponema. Cells where supernatants were positive for antibody toward parasites were transferred from these individual wells into a respective well of a 96-well tissue culture plate (Bellco) containing $1 \times 10^5$ BALB/c spleen feeder cells per well.

Cloned hybridomas were grown in ascites in BALB/c mice (6-10 weeks old). Mice were injected i.p. with 0.5 ml Pristane® (2,6,10,14-tetramethylpentadecane, Aldrich Chemical Co., Milwaukee, Wis.) on days 1 and 4. Five to ten days later, the mice were given an i.p. injection of $1 \times 10^6$ hybridoma cells in saline. Seven to ten days later, the ascites fluid was tapped from the peritoneal cavity.

D.    Characterization of Monoclonal Antibodies

    1.    Determination of monoclonals having
          specificity against T. pallidum.

Screening of hybrid clone culture supernatants for the presence of monoclonal antibodies directed against T. pallidum membrane antigens was performed using a micro ELISA technique. Freshly extracted T. pallidum organisms were purified by Methocel (Dow Chemical Co., Midland, MI)-Hypaque (Winthrop Laboratories, New York) gradient centrifugation as described in Alderete et al, Infect. Immun. 26:1048-56 (1979). Treponemal suspensions containing approximately $3 \times 10^8$ organisms per ml were washed once using phosphate-buffered saline (PBS; 137 mmol/l NaCl, 2.7 mmol/l KCl, 4.6 mmol/l $Na_2HOP_4$ and 1.5 mmol/l $KH_2PO_4$ pH 7.2) and centrifuged for 10 minutes at 17,500 x g. Pellets were resuspended to $3.5 \times 10^7$ treponemes per

ml PBS. Fifty microliters were distributed into each well of 96-well PVC microtiter plates (Dynatech Laboratories, Alexanderia, VA, USA), and suspensions dried in air at 37°C. Then 50 µl of 95% ethanol were added to each well, and plates were dried at 37°C and stored under desiccant at 4°C until used.

Purified <u>T. pallidum</u> proteins were diluted to 1 µg/ l in carbonate buffer, aliquoted (50 ng/well) on to 96-well microtiter plates, and incubated overnight at 4°C. If not used immediately, plates were washed once with PBS, filled with PBS-1% BSA and stored at -20°C.

A modified ELISA technique (Voller et al, <u>In</u>: N.R. Rose and H. Friedman, eds., <u>Manual of Clinical Immunology</u>, Washington, D.C., American Society for Microbiology, p.506 (1976)) was used for detecting antibodies to whole <u>T. pallidum</u> organisms. Briefly, after antigen coating of PVC microtiter plates, wells filled with PBS supplemented with 1% BSA were incubated for a minimum of two hours at 37°C. Plates were then washed three times with PBS, and 50 µl of test serum diluted with PBS-1% BSA were added to the designated wells. After incubation for one hour at 37°C the plates were washed four times with PBS. Fifty-microliter aliquots of alkaline phosphatase conjugated goat anti-rabbit IgG (Miles Laboratories, Elkhart, IN, USA) diluted 1/3000 in PBS-1% BSA were then added to each well. Plates were incubated for one hour at 37°C followed by sequential washing with PBS (three times) and distilled $H_2O$ (twice). Finally, 50 µl of 1 mg/ml disodium p-nitrophenyl phosphate (Sigma) prepared in diethanolamine buffer were added to each well, and the plates incubated for 30 minutes at 37°C. Antibody reactivity was measured by optical density readings at 405 nm using a MicroElisa Reader (Dynatech).

Microtiter wells in which the absorbance was at least twofold greater than background levels of absorbance obtained with antigen-free control wells were scored as positive for the presence of antibodies directed against T. pallidum organisms.

Of the approximately 1500 wells showing positive clone growth, 54 clones were reactive against whole T. pallidum organisms (ELISA values greater than 0.150).

    2.    Determination of monoclonals having specificity
    for antigenic determinants common to the treponemes
    pathogenic to humans and lacking specificity to
    treponemes nonpathogenic to humans.

The 54 monoclonals showing reactivity against T. pallidum whole organisms were next screened for reactivity against sonicated treponemes, namely T. pallidum and T. pertenue, the respective pathogenic agents for syphilis and yaws; T. hyodysenteriae and T. phagedenis biotype Reiter, two treponemes nonpathogenic to humans.

Sonicated treponemal antigens of each treponeme were prepared by suspending purified treponemes in carbonate buffer (pH 9.6; 1.59g $Na_2CO_3$ and 2.93g $NaHCO_3$ per liter). Sonification was accomplished by 6 15-second bursts with 45-second intermittent incubations on ice (Sonifier Cell Disruptor, Model W 140D, Branson Sonic Power Co.).

Each of the respective sonicated treponemal protein mixtures was coated onto microtiter wells as described above but at a concentration of 1 µg protein per well.

Of the 54 monoclonals selected for continued screening, the monoclonal antibodies produced therefrom were purified as follows. Six milliliters of mouse ascites fluid were diluted 1:5 with 0 01 M phosphate buffer pH 7.2 and applied to a 1.5 x 9 cm bed of protein A-Sepharose. Four ml fractions were collected, and the absorbance at 280 nm of each was monitored until the background was less than 0.02. The eluting buffer was then switched to pH 2.8 glycine hydrochloride. Those fractions containing $A_{280}$ absorbing material were pooled and dialyzed in a 2 step process, first against 50 mM citrate, 50 mM phosphate, 0.15 M NaCl, pH 5.5 and finally against PBS. The purified monoclonal antibodies were then diluted to 1 mg/ml in PBS and stored at -70°C in small aliquots until use.

Next the micro ELISA procedure as described previously was repeated to determine which monoclonal antibodies if any would cross-react with protein antigenic determinants common to the pathogenic treponemes yet not react with the treponemes nonpathogenic to man. Of the 54 monoclonal antibodies tested, 4 were directed against the pathogenic T. pallidum and T. pertenue treponemes which are pathogenic to man and not reactive with the treponemes which are not pathogenic to man. Table I describes the results.

Table I:  Reactions of monoclonal antibodies to various treponemal sonciates

|  | | Sonicated Treponemal Antigens | | | |
|---|---|---|---|---|---|
| Monoclonals | | | | | |
| | Dilution | T. pallidum | T. pertenue | T. hyodysenteriae | T. phagedenis |
| 11F$_2$ | 1:100 | 0.268 ± 0.015 | 0.198 ± 0.107 | 0.100 ± 0.007 | 0.042 ± 0.014 |
| 13F$_3$ | 1:100 | 0.394 ± 0.037 | 0.316 ± 0.149 | 0.049 ± 0.012 | 0.044 ± 0.009 |
| 23C$_9$ | 1:100 | 0.427 ± 0.047 | 0.382 ± 0.006 | 0.028 ± 0.005 | 0.018 ± 0.005 |
| 20G$_{11}$ | 1:100 | 0.271 ± 0.029 | 0.282 ± 0.035 | 0.054 ± 0.008 | 0.049 ± 0.002 |

3.    Determination of monoclonals having specificity against the immunodominant polypeptide P6

On further testing of the above four monoclonals, (11F$_2$; 13F$_3$;  23C$_9$; and 20G$_{11}$) it was shown that three of the monoclonals (11F$_2$; 13F$_3$ and 23C$_9$) produced antibodies having specificity for the immunodominant polypeptide P6 common to the pathogenic treponemes  T. pallidum and T. pertenue.  According to procedures described in Alderete et al, "Analysis of Serum IgG against Treponema pallidum Protein Antigens in Experimentally Infected Rabbits". British Journal of Veneral Disease 57:302-8 (1981) (incorporated herein by reference) the four tested monoclonal antibodies reacted with T. pallidum proteins as noted in Table II.  The treponemal component, P6, is a surface protein of approximately 42,500 daltons (molecular weight) and is one of the immunodominant envelope proteins
of virulent treponemes based upon the immunologic response
of infected humans and rabbits.

Table II  Rection monoclonal antibodies to T. pallidum proteins
eluted from SDS-polyacrylamide gels

| Monoclonals | Dilution | Control | P5,6 | P1,2,3,4 |
|---|---|---|---|---|
| | | | T. pallidum proteins | |
| $11F_2$ | 1:100 | $0.005 \pm 0.034$ | $0.138 \pm 0.047$ | $0.034 \pm 0.019$ |
| $13F_3$ | 1:100 | $0.003 \pm 0.005$ | $0.260 \pm 0.075$ | $0.001 \pm 0.004$ |
| $23C_9$ | 1:100 | $0.002 \pm 0.003$ | $0.244 \pm 0.033$ | $0.030 \pm 0.024$ |
| $20G_{11}$ | 1:100 | $0.004 \pm 0.039$ | $0.075 \pm 0.073$ | $0.042 \pm 0.011$ |

Western blot analysis of monoclonal antibody $13F_3$ against
various solubilized proteins of treponemes:  T. pallidum, T.

Western blot analysis of monoclonal antibody $13F_3$
against various solubilized proteins of treponemes:  T.
pallidum, T. pertenue, T. hyodysenteriae and T. phagedenis
biotype Reiter confirmed the observation that $13F_3$ mono-
clonal antibody was reactive with the immunodominant
protein P6 possessed by T. pallidum and T. pertenue but
absent from the other two treponemes tested.

To date, Applicant has deposited with the American
Type Culture Collection, Rockville, Maryland the hybrid
cell lines corresponding to clone $11F_2$ (ATCC accession
number HB 8396) and clone $13F_3$ (ATCC accession number
HB 8395).

E.  Detection of Antigen from Infected
    Tissues With Monoclonal Antibody 13F$_3$

In order to determine the efficacy of antigen detection of monoclonal antibody 13F$_3$ against T. pallidum, polyvinylchloride (PVC) microtiter plate wells (Dynatech, Immulon II plates) were coated with 1, 10, and 100 µg protein concentrations of ascites fluid containing the 13F$_3$ monoclonal antibody. This was accomplished using standard procedures with carbonate buffer.  Thus, the orientation of the 13F$_3$ antibody is that of an attractor molecule for specific binding of treponemal antigen in body fluids or tissues of infected individuals.  After overnight coating of microtiter plate wells, the plates were rinsed with PBS, allowed to dry at room temperature and stored at 4°C until use.  No loss of reactivity has been detected in plates stored for several weeks.  For the diagnostic purposes described in this application, only plates with antibody prepared the previous day were used.

Coated plates were then treated with PBS-1% BSA to tie up all nonspecific sites.  After a 2 hour incubation at 37°C, the plates were washed at least three times with PBS and to respective wells were added 100 µl of a freshly prepared homogenate [obtained as described below] of i) normal tissue from an uninfected rabbit, ii) tissue from the shaved back of a rabbit inoculated with T. pallidum and iii) T. pallidum organisms, as a positive control to make sure the antibody was reactive with treponemal components.  After a 2 hour incubation at 37°C, the wells were again washed as above and incubated with normal mouse serum as a control or pooled sera from mice infected with T. pallidum.  These sera were previously examined for their reactivity toward outer envelope proteins of T. pallidum using radioimmunoprecipitation techniques, and syphilitic mouse serum was found to possess high-titered antibody to key surface immunogens. Wells were treated with these antibody reagents for 60 minutes, washed, and incubated further at 37°C after addition of alkaline-phosphatase conjugated goat antimouse immunoglobulin (IgG fraction). After 60 minutes, wells were again washed with PBS and substrate added. Plates were incubated for 60 minutes further prior to reading of individual wells on a Dynatech microtiter plate scanner at 405 nm. (It is noteworthy that serum from syphilitic humans or rabbits could also be employed for the above assay, with slight modification. In this case, mouse serum reagents were used because of the lack of reactivity in control mice and the high-titered IgG found in syphilitic mouse sera). Results demonstrated the successful detection by $13F_3$ monoclonal antibody of treponemal antigen from infected rabbit lesions which develop seven days after challenge with 0.1 ml containing $10^6$ organisms. As expected, for each concentration of ascites coated onto PVC plates, the $13F_3$ monoclonal readily detected T. pallidum antigen from a homogenate of purified whole organisms. No reactivity was observed when an equivalent homogenate of control unifected tissue was employed for experiments performed identically.

A skin punch used in dermatology laboratories for obtaining tissue biopsies was used in order to obtain skin samples from rabbits as indicated above. Samples from two elevated lesions with a surface area of approximately 75 $mm^2$ and containing skin and intradermal tissue were minced and resuspended to 2 ml in PBS containing 1 mM phenylmethylsulfonylchloride (PMSF) (a nonspecific protease inhibitor) and 0.05% Zwittergent 3-12 detergent. This material was then homogenized using a Dounce homogenizer with a teflon pestle rotating at 100 revolutions per minute on a rotary motor. After approximately fifty strokes, the homogenate was transferred to microfuge tubes and centrifuged at 10,000 x g for 5 minutes. Supernatant was used as antigen source for the enzyme-linked immunosorbent assay described above. T. pallidum organisms were homogenized similarly by resuspending $10^{10}$ organisms previously washed well in PBS in 1 ml PBS-1 mM PMSF containing 0.05% Zwittergent 3-12 detergent. The suspension was homogenized with 10 strokes using a standard glass pestle, tightfitting homogenizer. Soluble material was centrifuged (100,000 x g) to remove insoluble debris, and supernatant added to wells for ELISA.

\*    \*    \*

While the compositions and methods of this invention have been descibed in terms of preferred embodiments, it will be apparent to those of skill in the art that various changes may be made in the compositions and methods disclosed, without departing from the scope of the invention, which is defined by the following claims:

-20- 0140689

CLAIMS:

1. A hybridoma consisting essentially of a continuous hybrid cell line which produces monoclonal antibody reactive against an antigenic determinant common to the human pathogenic treponemes but unreactive against the human nonpathogenic treponemes, which cell line is formed as a fusion between a myeloma cell and a lymphocyte immunized against human pathogenic treponemes.

2. The hybridoma of claim 1 wherein the hybrid cell line is a cell hybrid of a mouse lymphocyte immunized against human pathogenic treponeme antigen, fused to a mouse myeloma cell.

3. The hybridoma of claim 1 wherein the continuous hybrid cell line is a cell hybrid of a BALB/c mouse immune spleen cell immunized against human pathogenic treponeme antigen, fused to a mouse myeloma cell.

4. The hybridoma of claim 1 wherein the hybrid cell line is a cell hybrid of a mouse lymphocyte immunized against human pathogenic treponeme antigen, fused to a SP2/0 hybridoma cell.

5. The hybridoma of claims 1, 2, 3, or 4 wherein the human pathogenic treponeme antigen is a _Treponema pallidum_ antigen.

6.    The hybridoma of claims 1, 2, 3, or 4 wherein the human pathogenic treponeme antigen is P6 immunodominant protein.

7.    The hybridoma of claim 1 wherein the hybrid cell line is hybridoma clone ATCC deposit HB 8395 or HB 8396.

8.    A composition consisting essentially of monoclonal antibody having specificity against an antigenic determinant common to the human pathogenic treponemes but lacking specificity for the human nonpathogenic treponemes.

9.    The composition of claim 8 wherein the monoclonal antibody has specificity for an antigenic determinant common to T. pallidum and T. pertenue but lacking specificity to the treponemes not pathogenic in man.

10.    The composition of claim 8 wherein the monoclonal antibody has specificity for P6 immunodominant protein.

11.    The composition of claim 8 wherein the monoclonal antibody is produced from hybridoma clone ATCC deposit HB 8395 or HB 8396.

12.    A method of diagnosing human pathogenic treponeme infection in a host comprising:

        contacting a physiological sample from the host
            together with a composition of monoclonal

0140689

antibody having specificity for an antigenic determinant common to human pathogenic treponeme but lacking specificity for human nonpathogenic treponemes;

measuring the immunological binding reactivity between antigenic material of the physiological sample and the monoclonal antibody.

13. The method according to claim 12 wherein the physiological sample is serum.

14. The method according to claim 12 wherein the physiological sample is infected tissue.

15. The method according to claim 12 wherein the monoclonal antibody has specificity for an antigenic determinant common to T. pallidum and T. pertenue but lacking specificity to the human nonpathogenic treponemes.

16. The method according to claim 12 wherein the monoclonal antibody has specificity for P6 immunodominant protein.